# EUROPEAN PATENT SPECIFICATION

(11) **EP 4 528 270 B1**
(45) Date of publication and mention of the grant of the patent: **02.12.2026**
(21) Application number: 23198980.7
(22) Date of filing: 22.09.2023
(51) Int. Cl.: G01N 33/15

(54) **HEATING MODULE FOR HEATING A DISSOLUTION MEDIUM OF A DISSOLUTION TESTING APPARATUS**
HEIZMODUL ZUM ERWÄRMEN EINES AUFLÖSUNGSMEDIUMS EINER AUFLÖSUNGSPRÜFVORRICHTUNG
MODULE DE CHAUFFAGE POUR CHAUFFER UN MILIEU DE DISSOLUTION D'UN APPAREIL D'ESSAI DE DISSOLUTION

(43) Date of publication of application: 26.03.2025
(73) Proprietor: SOTAX AG, 4147 Aesch (CH)
(72) Inventor: Benz, Rolf, Aesch 4147 (CH); Gasser, Stefan, 4147 Aesch (CH); Kazmierczak, Damian, 4147 Aesch (CH); Hänggi, Steven, 4147 Aesch (CH); Kalbermatten, Gilles, 4147 Aesch (CH)
(74) Representative: Braunpat AG

(56) References cited:
- CN-A- 105 784 953
- CN-A- 113 189 286
- US-B1- 10 732 080
- US-B2- 10 940 480
- US-B2- 7 332 128
- ANONYMOUS: "British Pharmacopoeia 2022 - Volume 5 - Appendix XII", 1 January 2022 (2022-01-01), pages A392 - A417, XP093129868, Retrieved from the Internet <URL:https://vnras.com/wp-content/uploads/pdf/British-Pharmacopoeia-2022-Vol-5.pdf> [retrieved on 20240209]
- PRABHU NAMITA B. ET AL: "Comparison of Dissolution Profiles for Sustained Release Resinates of BCS Class I Drugs Using USP Apparatus 2 and 4: A Technical Note", AAPS PHARMSCITECH, vol. 9, no. 3, 18 June 2008 (2008-06-18), US, pages 769 - 773, XP093129848, ISSN: 1530-9932, DOI: 10.1208/s12249-008-9110-4

## Description

### Technical Field

The invention relates to dissolution medium heating modules for a dissolution testing apparatus, particularly to dissolution medium heating modules, integrated into a flow-through cell instrument or apparatus used for drug release testing and dissolution testing.

### Background of the invention

Dissolution testing is a compendial in vitro test for example used in pharmaceutical research and development to characterize the release rate of an Active Pharmaceutical Ingredient (API) from a formulated dosage form, under reproducible conditions, in correlation with its in vivo performance. Once an innovator product dossier has been approved by the addressed market authorities, dissolution testing is then used to assess batch-to-batch consistency of the manufactured product according to its submitted specification and according to general testing conditions described by authorities. Dissolution is also used by generic companies to establish the bioequivalence of their products, without redoing clinical studies initially done by the innovator: doing so they obtain a biowaiver.

Pharmaceutical formulations include granules, tablets, capsules, gels, creams, ointments, suspensions, suppositories, patches, microspheres, implants and other long acting injectables as micro and nano suspensions and others. "Dissolution testing" is a term used for solid dosage forms and the more general term "Drug release testing" is used for other dosage forms, including coated medical devices. Outside of the pharmaceutical segment, dissolution testing apparatuses are also used in applications such as nicotine release from smokeless tobacco products and for persistence tests of stone wool fibers in lung fluids. The first interest of a dissolution testing apparatus in form of a flow-through cell dissolution instrument lies in its flexibility as the cell and its inside arrangement can be adapted to the tested formulation structure and challenges.

Dissolution testing modalities for solid dosage forms and apparatus involved are described in a general dissolution chapter harmonized between European, US and Japanese Pharmacopeias: the USP <711>. A flow-through cell dissolution is also described as method 6 in the Chinese Pharmacopeia.

Standardized methods use for example a paddle apparatus for stirring substances in a container as for example described in EP 0746759 A1, a basket apparatus for holding the substance in a basket as for example described in US 5816701 A, or a flow-through apparatus providing dissolution medium flowing through a cell containing the substance as for example described in WO 2013/003518 A1 or in Volume 5 of the British Pharmacopoeia of 2022 (ISBN 978 011 3230 877). The dimensions and tolerances of such apparatuses are specified precisely. Critical test parameters that have to be monitored periodically during use include for example volume and temperature of the dissolution medium, rotation speed and flow rate of medium.

The flow-through apparatus is used for a wide range of objects to be tested. The flow-through cell dissolution principle consists in flowing dissolution medium at defined temperature and flow rate onto a cell containing the dosage form to be tested. The total volume of dissolution medium is based on the solubility of the active pharmaceutical ingredient respectively substance, to be dissolved and the test duration is ideally correlated to the in vivo performance of the tested product. Therefore, the temperature of the media (from 32°C for transdermal products to 37°C for oral products and up to higher temperatures in the scope of accelerated testing) flowing into the cell has to be controlled all through the test. The cell temperature control and its efficiency are linked to two processes: the temperature control of the media flowing into the cell (at different possible flow rates as the flow rate is a method parameter) and the ambient temperature of the chamber the cell stays in.

The flow-through apparatus can be realized as an open system or a closed system as for example illustrated in EP 0049293 A1. The open system uses dissolution medium that is pumped, in all channels, from a reservoir through the cells and collected or analysed after the cells. The open system is for example recommended for poorly soluble drugs which require a high volume of media or for pH changes to be performed for example in order to mimic the Gastro-Intestinal tract. In a closed system, the media is pumped from one single reservoir per channel, through the cell and brought back to the reservoir. These individual loops for each channel allow an accumulation of dissolved active ingredient in the reservoirs which can be sampled or analysed.

In general, a dissolution testing apparatus is composed of multiple channels to allow at least the simultaneous testing of seven channels (six samples and a reference). Therefore, the seven channels have to be qualified in a documented procedure (Installation Qualification, Operational Qualification) prior to any use of the apparatus to prove that they allow an equivalent testing on all channels, as finally samples will be compared to specifications as they are representing the entire manufactured batch. The temperature in each cell (as the flow rate of media entering) has therefore to be accurate and consistent all over the test duration in order that the test only detects sample variations.

Historically, heating systems of dissolution testing apparatuses which guarantee the entrance of media at the right temperature are for example individual heating coils located in a unique temperature-controlled water bath. An individual channel cannot be individually calibrated nor regulated. Oftentimes, the cells are for example provided with heating systems comprising individual heating elements or heating units for each cell, as for example shown in WO 2010/059643. Further, an air stream or an air bath may be applied to each cell for tempering the cell and the dissolution medium, respectively, as for example shown in US 2015/0358587 A1. The cells can be associated to temperature sensing elements, which are connected to heat control systems for controlling the temperature in the cell.

Other known heating systems include a conduit for the dissolution testing medium which is spirally wound and guided through a heated water bath as shown in CN 105 784 953 A and Prabhu et al. (AAPS PharmSciTech 9-3/2008, doi: 10.1208/s12249-008-9110-4).

However, the known dissolution testing apparatuses oftentimes are slow or complicated in adjusting the temperature of the dissolution medium in the cells. Particularly, heating arrangements using a water bath are difficult to control in a short period of time. Further, heating elements applied to each individual vessel as disclosed in the state of the art require additional application and maintenance steps for preparing and performing the testing. Most apparatuses are designed as an open system or a closed system, and they are not set up to easily switch between an open and closed configuration. Such shortcomings impact efficient quality control, compliance monitoring and product optimization.

It is an object of the invention to provide a dissolution testing apparatus and a dissolution medium heating module for a dissolution testing apparatus, that enable fast and precise temperature adjustment of the dissolution medium, allow for individual temperature control of individual dissolution cells, provide a simple and flexible set up and ensure precise and reproducible testing.

### Summary of the invention

These and other objects, which will appear from the description below, are achieved by a dissolution medium heating module and a dissolution testing apparatus set forth in the appended independent claims. Preferred embodiments are defined in the dependent claims.

A dissolution medium heating module for a dissolution testing apparatus for dissolving a dissoluble substance in the dissolution medium according to the present invention comprises a heating chamber, which is connect or connectable, for example by a pump device, to a dissolution medium reservoir for receiving the dissolution medium, and at least one planar heating surface arranged at the heating chamber for heating the dissolution medium therein. The heating chamber is realized as a two-dimensional heating loop structure comprising a medium inlet at one end of the heating loop structure and a medium outlet at an opposite end of the heating loop structure for receiving and dispensing dissolution medium. Advantageously, the medium outlet can be connected directly to a dissolution cell of the dissolution testing apparatus, which is arranged for holding the dissoluble substance to be tested. The at least one planar heating surface of the dissolution medium heating module is provided adjacent to the heating loop structure for emitting and transferring thermal energy to the heating loop structure.

The two-dimensional loop design of the heating loop structure provides a very large surface area for the dissolution medium to interact with the thermal energy and a narrow heating space to be heated up compared to conventional dissolution medium containers. This design accelerates the heat exchange and reduces energy loss. The dissolution medium can quickly be heated to a predetermined temperature in the heating loop structure by the at least one planar heating surface while the dissolution medium is in flow from a medium reservoir to a dissolution cell of a dissolution testing apparatus. The medium flow or circulation to the cell is not interrupted for heating the dissolution medium and dissolution medium can be provided to the cell continuously with a predetermined temperature. The inventive dissolution medium heating module allows for fast and flexible supply of precisely tempered dissolution medium.

The dissolution medium reservoir is connectable to the heating loop structure by a dissolution medium conduit supplying the medium to the heating loop structure. The dissolution medium reservoir may be part of the dissolution testing system, for example provided in a dissolution module, extraction module or sample module of the apparatus, and can be connected to the dissolution medium conduit of the heating module.

The heating loop structure is designed as a medium conduit or pipe which is looping in one coordinate plane. The extend of the heating loop structure preferably covers the entire area of the at least one planar heating surface. The medium conduit/pipe can for example have a convoluted, wriggled, or serpentine looping layout, which optimizes the use of the plane area corresponding to the surface of the at least one planar heating surface. In one alternative of the heating module, there may be two planar heating surfaces arranged parallel to each other, which are enclosing the heating loop structure on both sides. The planar heating surface can be realized by a single heating element or more than one heating elements next to each other can be used on one side of the heating loop structure to cover the area of the structure.

The medium outlet of the heating loop structure can be connected to a cell inlet of a dissolution cell, for example using a medium outlet line and/or an in-flow conduit for providing tempered dissolution medium to the cell. Advantageously, the medium outlet of the heating loop structure is located in short distance to the inlet of the dissolution cell to avoid any heat loss while the dissolution medium flows through the medium outlet line and/or the in-flow conduit.

In one embodiment of the dissolution medium heating module according to the invention the planar heating surface is realized by heating plates, preferably ceramic plates, comprising interior heating coils. The heating coils are operated electrically, for example. Ceramic plates have a low expansion coefficient and high thermal conductivity, which allows for reproducible and fast heat transfer.

Further, a media selector can be connected to the medium inlet of the heating module, for example by the pump. The media selector provides various types of solvents in different amounts, which for example are specified in a test protocol for testing a specific dissoluble substance in correspondence to in vivo conditions. The media selector conveys the specified solvent and amount thereof to the dissolution medium conduit, for example through the pump, supplying medium to the heating loop structure, the medium outlet line leading to the cell.

In a further embodiment of the dissolution medium heating module according to the invention a fluid pump is arranged in flow direction before the heating loop structure for controlled discharge of dissolution medium to the heating loop structure and into the dissolution cell. Advantageously, the fluid pump is arranged between the medium reservoir and the heating loop structure to ensure precise administration of dissolution medium volumes. The fluid pump could also be designed as a pressure gauge for providing information about a fluid pressure in the heating loop structure or a clogging of any dissolution medium conduits.

In a still further embodiment of the dissolution medium heating module according to the invention at least one temperature sensor is provided at the heating loop structure, for example at the medium outlet and/or medium inlet. The temperature sensors provide instant temperature monitoring of the dissolution medium immediately before the dissolution medium enters the dissolution cell, which allows for precise dissolution testing if the release is temperature dependent.

Preferably, the dissolution medium heating module according to the invention comprises a control unit for controlling the temperature of the planar heating surfaces for example according to temperature measurements of the temperature sensors. Further, the control unit may control the pump operation and a pressure in the heating loop structure and/or in dissolution medium conduits like the medium outlet line and the in-flow conduit to the dissolution cell. The control unit may also control the flow rate of the dissolution medium to ensure constant and continuous medium input to the dissolution cell. Further, the control unit may control the type and the amount of solvent provided by the media selector.

According to a further aspect of the present invention a dissolution testing apparatus comprises at least one reservoir for a dissolution medium, which is in fluid communication with at least one dissolution cell holding a dissoluble substance to be tested, a pump device for adding dissolution medium from the at least one medium reservoir to the at least one dissolution cell, and at least one dissolution medium heating module as described above. The at least one reservoir is connected or is connectable to the pump device then to the medium inlet of the heating loop structure, and the medium outlet of the heating loop structure is connected or is connectable to the at least on dissolution cell. The connection for example is realized by a simple fluid conduit or can be established for example by valves or manifolds establishing a fluid communication. Advantageously, one dissolution medium heating module is provided for each cell of the dissolution testing apparatus. Due to the narrow construction design of the dissolution medium heating module several modules can be stacked adjacent to each other in a small space for serving several cells aligned next to each other.

Advantageously, the dissolution testing apparatus comprises several dissolution cells for simultaneous testing of several units of a dissoluble substance, which allows for fast and reproducible dissolution testing. The several dissolution cells are aligned in parallel and several heating modules, each associated to one cell, are aligned in parallel below the cells for providing upwards flows. Again, the narrow construction of the heating modules allows to provide an independent heating arrangement for the dissolution medium provided to each of the cells.

Preferably, the dissolution testing apparatus is designed for a closed loop configuration and/or an open loop configuration, like known for a USP 4 apparatus, wherein the at least one cell is a flow-through cell comprising a cell inlet for the dissolution medium and a cell outlet for a sample solution containing dissolution medium and dissolved substance. In the closed loop configuration, the sample solution can be returned as dissolution medium into the flow-through cell to accumulate dissolved substance in the reservoir. In
the open loop configuration, the sample solution is extracted in fractions and then transferred into sample vials for analytical testing or analysed on-line.

Advantageously, the heating loop structure of the at least one heating module is arranged in the dissolution medium flow provided by the pump device between the at least one reservoir and the at least one cell. That means the heating module is arranged in series in flow direction with the associated cell and, the heating loop structure establishes a section of a medium conduit from the reservoir to the cell. Additional fluid branches or fluid compartments, that need to merge into the medium conduit are avoided.

In one embodiment of the dissolution testing apparatus according to the invention the at least one heating module is detachably connected to the cell. Also, it could be detachably connected to the reservoir, if desired.The two-dimensional heating loop structure and the at least one planar heating surface are arranged in a heating module housing that comprises fluid connectors. The fluid connectors of the housing connect the medium inlet of the heating loop structure to a fluid connector in communication with the medium reservoir, and to connect the medium outlet of the heating loop structure to a fluid connector in communication with the inlet of the dissolution cell. The fluid connectors may for example connect with each other by a press fit, form fit, thread connection, bayonet connection or the like and detach by releasing the fit or connection. Thus, the heating modules can easily be detached from the dissolution testing apparatus for example to provide access to the interior of the apparatus, for maintenance or replacement of components.
medium outlet of the heating module and an outlet of the cell for bypassing the cell. The bypass conduit may be arranged in parallel to the dissolution cell and connected to the in-flow conduit entering the cell by a valve and to the out-flow conduit exiting the cell by a further valve. The bypass conduit helps to regulate the dissolution conditions in the dissolution cell and replaces the cell passage of the medium flow. Thus, the dissolution medium can cycle through the dissolution testing apparatus even without a dissolution cell in place. This by-pass helps in pre-test temperature control or UV-Vis on-line analytical baseline and at the end of the test to clean the system tubing.

In a further embodiment of the dissolution testing apparatus according to the invention a controller controls the operating conditions of the dissolution testing apparatus. The controller for example comprises a processing unit, a user interface and a control display. The controller for example receives measurement data from temperature sensors of the heating module, from further temperature sensors arranged at the dissolution cell or in a cell chamber accommodating the cells or arranged at the medium reservoir, from pressure sensors or gauges arranged in the dissolution medium conduits, particularly in the out-flow conduit of the heating module and the cell, from sample solution testing devices, like a UV-Vis spectrophotometer, or any other sensors or devices used in the dissolution testing apparatus. The control units of the dissolution medium heating modules can be integrated in the controller or be controlled by the controller. For example, the controller receives a testing protocol for testing a specific dissoluble substance and controls the operation of the dissolution testing apparatus according to requirements of the testing protocol.

In a still further embodiment of the dissolution testing apparatus according to the invention the dissolution cells are arranged in a cell chamber that serves as a heating channel. A heater is connected to the heating channel for heating air or another gas in the heating channel, while the dissoluble substance is exposed to the dissolution medium. The heating channel and the heater avoid a change of temperature of the dissolution medium after the dissolution medium has been heated and emitted from the heating loop structure into the dissolution cells. Thus, the test conditions in the dissolution cell can be held steady during the dissolution process.

The dissolution medium heating module and the dissolution testing apparatus comprising such dissolution medium heating modules improve the reproducibility of the assessment of dissolution qualities of dissoluble substance by improving the compliance to test requirements. They assist in providing consistent dissolution quality for dissolving substances, particularly of drug substances. Also, they provide a predictive measure of the efficacy of API substances and other drug-based applications.

### Brief description of the drawings

Preferred embodiments of the invention will be described in the accompanying drawings, which may explain the principles of the invention but shall not limit the scope of the invention. The drawings illustrate:
Fig. 1: an overview of operation modules of a dissolution testing apparatus according to the present invention,
Fig. 2: an inside view of a dissolution module of the dissolution testing apparatus of figure 1 comprising dissolution medium heating modules according to the present invention,
Fig. 3: a three-dimensional view of the dissolution medium heating module according to the invention,
Fig. 4a: a longitudinal view cut through the dissolution medium heating module of figure 3,
Fig. 4b: a cut through line B-B in Figure 4a of the dissolution medium heating module of figure 3,
Fig. 5: a schematical top view of a heating arrangement for heating a cell heating channel of a dissolution testing apparatus according to the present invention,
Fig. 6a -6c: illustrative views of three operating modes of the dissolution testing apparatus: testing mode, bypass mode and cleaning mode,
Fig. 7a & 7b: a side view and a schematical interior side view of a flow-through cell as used in a dissolution testing apparatus of the present invention, and
Fig. 8a & 8b: a schematical diagram of an open loop configuration and a closed loop configuration of a dissolution testing apparatus according to the present invention.

### Detailed description of a preferred embodiment

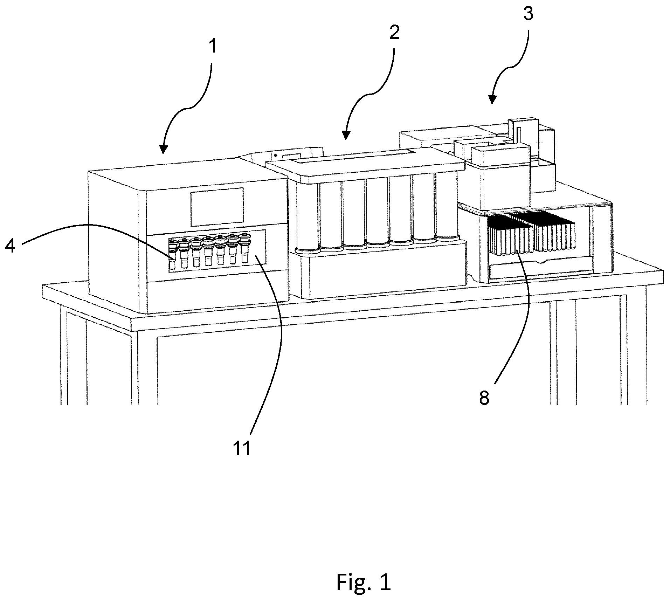

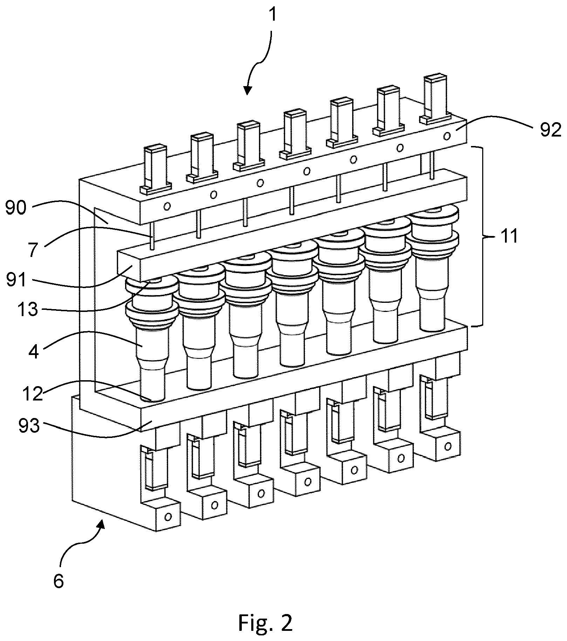

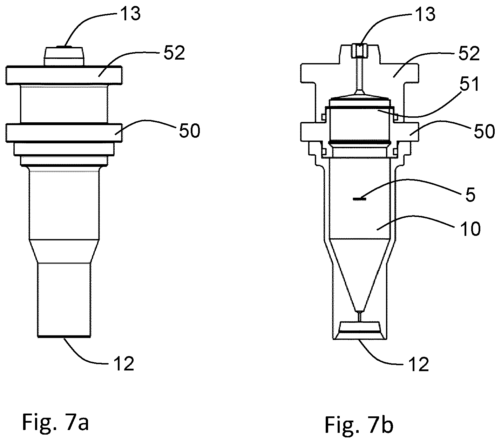

Figure 1 illustrates a setup of a dissolution testing apparatus as used in the present invention. The dissolution testing apparatus comprises a dissolution module 1, an extraction module 2 and a sample module 3. The dissolution testing apparatus can be operated in a closed mode configuration, or an open mode configuration as will be explained below. The dissolution module 1 accommodates several dissolution cells designed as flow-through cells 4, that are lined up in a cell chamber 11 of the dissolution module 1. Each cell is filled with a dissoluble substance 5 (see Figure 7a and 7b). The flow-through cells 4 are supplied with dissolution medium by dissolution medium heating modules 6 arranged below the cells in the dissolution module 1, as depicted in Figure 2. After passing the flow-through cells 4 the dissolution medium including dissolved substance exits the flow-through cells 4 as a sample solution containing dissolution medium and dissolved substance. The sample solution is transferred to the extraction module 2 via a sample solution conduit 7. In the extraction module 2 defined fractions of the sample solution are extracted from the sample solution conduit 7 into sample vials 8. Depending on the operation mode, the sample fractions in the sample vials can be tested and returned to their respective cell or the sample vials can be transferred to the sample module 3 for testing.

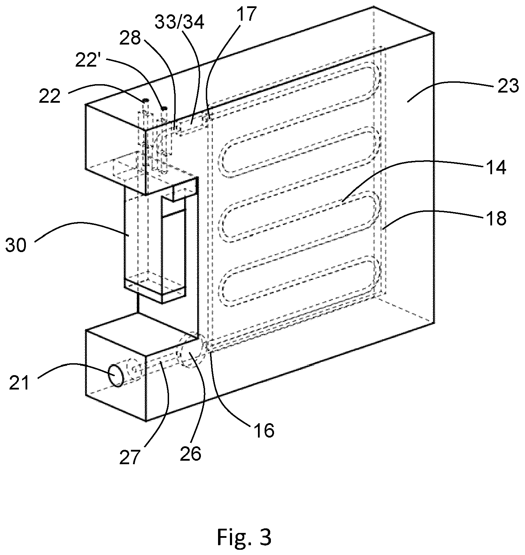

As can be seen in Figure 2, showing the interior of the dissolution module 1 of the dissolution testing apparatus according to the invention, the cell chamber 11 is realized above the dissolution medium heating modules 6 for heating the dissolution medium before the dissolution medium enters the cells 4. An assembly structure 90 in form of a mounting plate is arranged inside the dissolution module 1. On a front side, the assembly structure 90 comprises a cell rack holder 91 for holding the flow-through cells 4 upright and aligned along a longitudinal axis, a connector bar 92 comprising connector ports for connecting cell outlets 13 to the sample solution conduits 7, and a connector platform 93 for supporting the dissolution medium heating modules 6 below the platform and the flow-through cells 4 on top of the platform. The connector platform 93 comprises connector ports for connecting medium outlet lines 28 of the heating modules 6 to cell inlets 12 (see Figure 3). The connector bar 92 and the connector platform 93 extend distanced from each other and basically perpendicular from the assembly structure 90. Thus, the assembly structure 90, the connector bar 92 and the connector platform 93 create the cell chamber 11.

As shown in Figure 2 seven flow-through cells 4 are positioned in the cell chamber 11 such that a cell volume 10 (see Figure 7b) is fully exposed and accessible from all sides through walls of the flow-through cells, for example for visual inspection and interaction with conditions of the ambient environment present in the cell chamber 11. The cells comprise the cell inlet 12 at a bottom end and the cell outlet 13 at an upper end. The cell outlets 13 are in fluid communication with sample solution conduits 7, which deliver the sample solution to the extraction module 2. The bottom end of the flow-through cells 4 is positioned on the connector platform 93 for fluid connection to the dissolution medium heating modules 6 arranged below the connector platform 93 in the dissolution module 1. Each flow-through cell 4 is associated to one dissolution medium heating module 6, respectively, wherein the dissolution medium heating modules 6 are aligned next to each other below the cells.

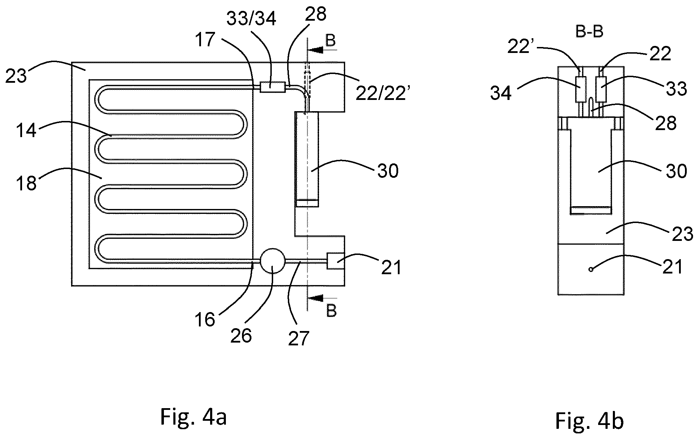

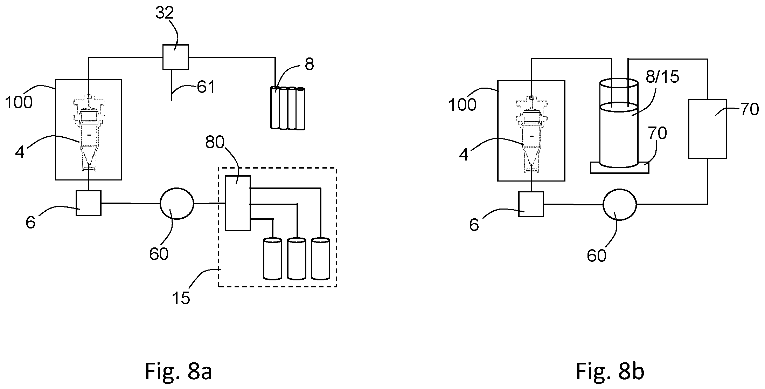

Figures 3 and 4 show the dissolution medium heating module 6 according to the present invention. The heating module comprises a heating chamber, which is realized as a two-dimensional heating loop structure 14. The heating loop structure 14 is for example designed as a medium conduit that loops in one plane for example in a serpentine pattern. The heating loop structure 14 is connected to a dissolution medium reservoir 15 (see Figures 8a and 8b) via a pump device 60 for receiving a dissolution medium. The heating loop structure 14 comprises a medium inlet 16 at one end of the heating loop structure 14 and a medium outlet 17 at an opposite end of the heating loop structure 14 for receiving and dispensing dissolution medium.

A planar heating surface in form of a heating plate 18 is arranged adjacent to the heating loop structure 14 for heating the dissolution medium in the heating loop structure 14. For example, the heating plate 18 comprises a size that covers an area of the two-dimensional heating loop structure. Alternatively, two or more heating plates or other flat heating elements or plates could be used to cover the heating loop area. The heating plate 18 is for example a ceramic plate that comprises interior heating coils. Also, silica materials or metal could be used for the planar heating surface.

The heating module receives dissolution medium from the external dissolution medium reservoir 15, which is for example located in the dissolution module 1 or in the extraction module 2 of the dissolution testing apparatus.

In the example embodiment shown in Figures 3 and 4, the dissolution medium heating module 6 comprises two housing half shells, which enclose the heating loop structure 14 and the heating plate 18 in an assembled state. One or both of the housing half shells can comprise a recess structure that corresponds to the two-dimensional heating loop structure 14 for providing, respectively receiving, the heating loop structure therein. Further, the housing half shells have an inlet through hole 21as well as a first and second outlet through hole 22 and 22' serving as medium conduits to and from the medium inlet 16 and the medium outlet 17 of the heating loop structure 14. The two housing half shells can be screwed together to realise a heating block 23 of the heating module 6.

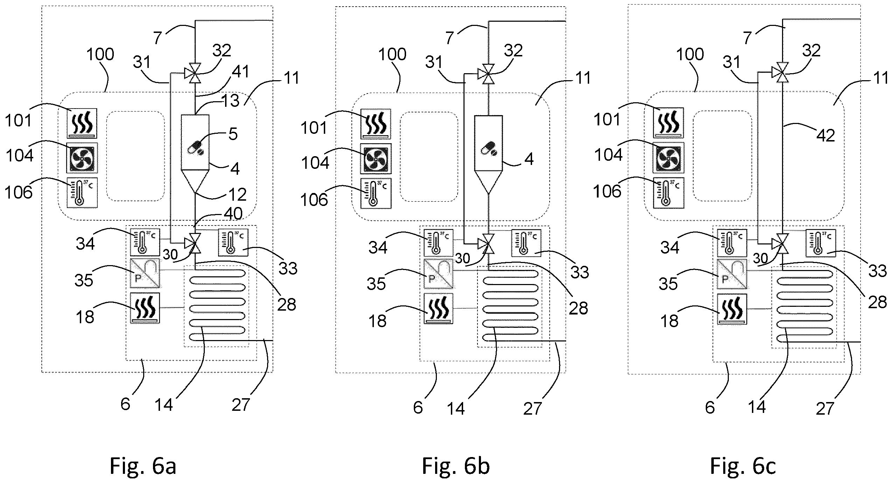

The dissolution medium heating module 6 comprises a pressure sensor 26 in a medium inlet line 27 for monitoring a pressure in the heating loop structure 14. The medium inlet line 27 is provided by the through passage of the through hole 21 and is detachably connected to the external dissolution medium reservoir 15 for example by an input port at the through hole 21. The medium outlet 17 merges into a medium outlet line 28, which enters a valve unit 30 (see Figures 6a - 6c), which can be attached to the housing. The valve unit 30 switches between parallel through passages provided by the first through hole 22 and the second through hole 22'. The first through hole 22 is connectable to the cell inlet 12 to provide tempered dissolution medium to a flow through cell 4. The second through hole 22' is connectable to a bypass conduit 31 for bypassing the cell. Each of the through holes 22 and 22' comprise a temperature sensor 33 and 34, respectively, for monitoring the temperature of the dissolution medium exiting the heating loop structure 14. The temperature sensors 33/34 and the pressure sensor 26 can be placed in the medium inlet line 27 and the through holes 22/22' when the two housing half shells are assembled to realise the heating block 23.

In an assembled state of the dissolution medium heating module 6, wherein the housing half shells are put together to form the heating block 23 the heating module comprises a slim design, wherein a thickness measured across the two half shells is only approximately half of a length or a hight of the heating module. Preferably, the thickness is only about a 1/3, more preferably only about 1/4, of the length or height. The slim design allows to provide individual dissolution medium heating modules 6 for each of the flow-through cells 4 of the dissolution testing apparatus without occupying a large space.

As will be explained in more detail for Figure 8a, a media selector 80 may be connected to the medium inlet line 27 for selectively adding a solvent to the dissolution medium before the medium enters the heating loop structure, as mentioned above. The medium can be provided to the inlet line 27 by a pump device 60.

Further, the dissolution medium heating module 6 may comprise control sensors for monitoring and controlling the operation of the heating module 6 and the dissolution testing apparatus. For example, at least one temperature sensor may be provided for monitoring a temperature of the dissolution medium along its flow through the heating module. A temperature sensor may for example be provided at the medium inlet line 27. The temperature sensors 33 and 34 are provided in the through holes 22 and 22'. Further, at least one pressure gauge, like the pressure sensor 26, may be arranged for example in the medium inlet line 27 and/or the medium outlet line 28, which could provide pressure measurements for controlling the medium input. A control unit controls the at least one heating plate 18 and the pump operation, as will be explained below.

As illustrated in Figure 2, the dissolution medium heating modules 6 are detachably mounted in the dissolution module 1 and the medium outlet line 28 of the heating loop structure 14 is detachably connected to the cell inlet 12. Thus, the heating module 6 can be replaced easily for maintenance.

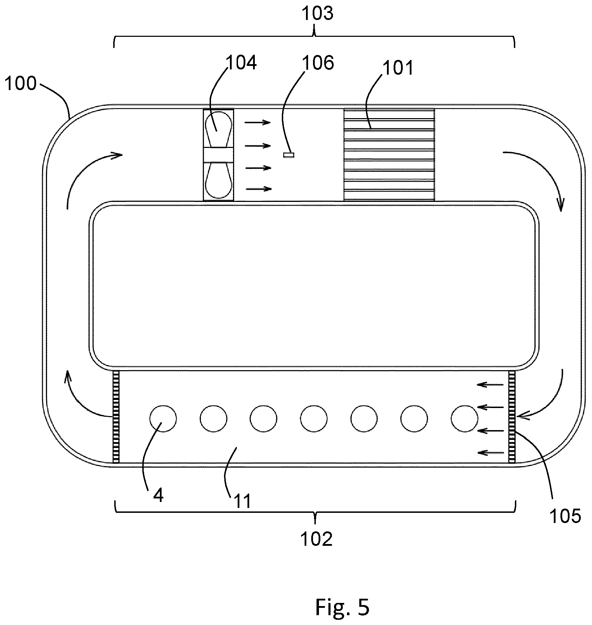

Figure 5 shows a heating arrangement for heating the several dissolution cells 4 accommodated in the cell chamber 11 of the dissolution module 1. In the shown example, the cell chamber 11 is part of a heating channel 100 common for all cells 4. A heater 101 is arranged in the heating channel 100 for heating air or another gas in the heating channel 100. A ventilator 104 is arranged next to the heater 101 in the heating channel 100 for creating a heated air stream in the heating channel and along the flow-through cells 4. In the shown example, the heating channel 100 is realized as a heating channel loop comprising a first elongated section 102 accommodating the cell chamber 11 with the several flow-through cells 4 and a second elongated section 103 accommodating the heater 101 and the ventilator 104. The first elongated section 102 and the second elongated section 103 are connected by curved sections to realize the heating channel loop. The several flow-through cells 4 are aligned along a longitudinal axis of the first elongated section 102.

The heater 101 comprises a plurality of flat heating elements, like heating plates, arranged in the heating channel 100 along a longitudinal axis of the heating channel. The air stream also flows along the longitudinal axis and can easily pass by the flat heating elements to be heated. A set of guiding plates 105 is located upstream of the several cells 4 for supporting a laminar flow along the cells in the first elongated section 102 of the heating channel 100. Further guiding plates may be located in curved section of the heating channel for supporting a laminar flow along the heater 101 and the ventilator 104.

Further, the heating channel 100 comprises fluid passages (not shown in Figure 5) for dissolution medium conduits in fluid connection with the flow-through cells 4, for example for the medium outlet line 28 of the heating modules 6 that is connected to the cell inlet 12 for transmitting dissolution medium into the cell 4 and for the sample solution conduit 7 for transmitting sample solution from the cell outlet 13 to the extraction module 2.

The common heating channel comprises at least one temperature sensor 106 (see Figures 6a - 6b) in the first elongated section 102 of the heating channel 100, which houses the several flow-through cells 4. Further temperature sensors can be located at or near at least one of the flow-through cells 4 and in the area of the heating channel accommodating the ventilator 104. A control unit at the heating channel and/or a controller of the dissolution testing apparatus may receive information data of the temperature sensors for controlling the heater 101 and/or the ventilator 104 according to a desired temperature in the heating chamber 11.

As can be seen in Figure 2, showing the interior of the dissolution module 1 of the dissolution testing apparatus according to the invention, the heating chamber 11 of the heating channel 100 is realized above the dissolution medium heating modules 6 for heating the dissolution medium before it enters the cells 4. The assembly structure 90, the connector bar 92 and the connector platform 93 create the cell chamber 11 and the heating channel 100, respectively. The heater and the ventilator may be located on a back side of the assembly structure 90. The assembly structure 90 with the above-mentioned assembled components are mounted in a housing of the dissolution module 1.

In the area of the elongated section 102 of the heating channel 100, representing the sample chamber 11, the housing comprises an opening along the length of the sample chamber 11 and a cover (not shown) for closing the opening. The opening and the cover provide access to the cell chamber 11 for positioning the flow-through cells therein and connecting them to the connection ports. The cells are surrounded by the heated air stream and the flow of the dissolution medium through the cells is perpendicular to the heat stream in the heating channel, which allows for uniform exposure to the heated air.

In the dissolution testing apparatus of the present invention, the dissolution medium is quickly and effectively heated to a desired temperature by the heating modules 6, while the dissolution medium is in flow to the flow-through cells 4. The heating loop structures 14 of the heating modules 6 are arranged in-line of the dissolution medium flow between the dissolution medium reservoir 15 and the flow-through cells 4. That means the heating modules 6 are arranged in series with their associated cells 4. The heating channel 100 is provided primarily to keep the temperature of the dissolution medium at the temperature achieved by the heating modules while the medium flows through the cells 4. This set up ensures a precise continuous flow of medium through the cells with a predetermined constant temperature and enables flexible and predictive dissolution testing of dissoluble substances.

Figures 6a to 6c describe different operations of the dissolution testing apparatus according to the present invention, schematically illustrating a flow of the dissolution medium provided by the dissolution medium heating module 6 and the cell chamber 11. As mentioned before, dissolution medium is provided by the medium inlet line 27 and enters the heating loop structure 14, wherein it is heated by the heating plate 18 while flowing through the structure. The temperature sensor 33 measures a temperature and a pressure gauge 35 measures a pressure of the dissolution medium in the medium outlet line 28. The bypass valve 30 arranged in the medium outlet line 28 dispenses the dissolution medium ether to an in-flow conduit 40 connected with the cell inlet 12 or to the bypass conduit 31. The temperature sensor 34 measures a temperature in the bypass conduit 31. In the cell chamber 11, respectively the heating channel 100, the dissolution medium is already heated by the heating module 6, flows through the flow-through cell 4 and dissolves the dissoluble substance 5 therein, which results in a sample solution containing dissolution medium and a portion of the substance dissolved therein. The sample solution exits the flow-through cell 4 through the cell outlet 13 into an out-flow conduit 41. The out-flow conduit 41 and the bypass conduit 31 end in the bypass valve 32, from where medium flow continues into the sample solution conduit 7 towards the extraction module 2 and/or sample module 3.

In Figure 6a the dissolution testing apparatus is operated in a testing mode, wherein heated dissolution medium is transferred from the dissolution medium heating module 6 into the flow-through cell 4 in the heating channel 100. The temperature in the flow-through cell 4 is kept or adjusted in the heating channel, while dissoluble substance dissolves into the dissolution medium. A sample solution is emitted into the sample solution conduit 7. The sample solution can be tested while flowing through the sample solution conduit 7, for example using optical analysis testing, or is directed to the extraction module 2 for extraction into sample vials 8 and testing.

In Figure 6b the dissolution testing apparatus is operated in a bypass mode, wherein the split valve 30 directs dissolution medium from the medium outline line 28 and the through hole 22' of the heating module 6 into the bypass conduit 31. This may be the case for example, if the flow-through cell is clogged, or in case of any other failure of the dissolution testing apparatus. From the bypass conduit 31 the bypass valve 32 directs the dissolution medium into the sample solution conduit 7, from where the dissolution medium may be discarded or returned into the dissolution medium cycle.

In Figure 6c the dissolution testing apparatus is operated in a cleaning mode, wherein the flow-through cell has been removed from the cell chamber 11. The dissolution medium or a cleaning medium may be used for cleaning the conduits of the dissolution testing apparatus. The medium runs through the heating module 6 and the bypass valve 30 directs the medium through the bypass conduit 31 and a replacement conduit 42 replacing the in-flow conduit 40, the flow-through cell 4 and the out-flow conduit 41. The bypass valve 32 directs the medium into the sample solution conduit 7.

Figures 7a and 7b show a flow-through cell 4 as it can be used for the present invention in an assembled top view and a schematical interior view. The flow-through cell 4 comprises a cell volume 10 having a generally cylindrical shape and a predefined size. The cell volume 10 is tapered towards the bottom end, which comprises the cell inlet 12, and is open at an upper end. A dissoluble substance 5 is placed in the cell volume 10. Holding structures like a basket, a bead bed or similar may be used inside the cell volume to place the dissoluble substance distanced from the bottom if required by a used testing method or a shape of a dissoluble substance. A closing ring 50 with a central through passage and a holding frame for a filter 51 is placed on the upper opening of the cell volume 10. The filter 51 can be selected according testing requirements for the tested dissoluble substance. A cap 52 comprising the cell outlet 13 is mounted on the closing ring 50 to close the cell volume 10 and keep the filter 51 in place. Alternatively, the closing ring 50 and the cap 52 can be realized a single piece, wherein the filter 51 can be arranged in the single piece before mounting the piece on the cell volume.

The cell volume 10 is made for example of glass or plastic material that is inert with respect to the dissoluble substance and comprises transparent walls for observing the dissolution process. The cell volume 10 does not need to be placed in additional containers as known from the prior art for providing heating means at the cell. The flow-through cell 4 comprises a simple design and allows for easy preparation of dissoluble substances for testing.

Advantageously, the closing ring 50 or the cap 52 may comprise an electronic chip, a temperature sensor, a positioning sensor, a flow meter, and other sensor means. The electronic chip is designed for wireless communication with the controller of the dissolution testing apparatus and transmits information data of the temperature at the dissolution volume, the cell position, the flow through the cell, etc.. The data can be used for controlling the dissolution medium heating modules 6, the heater 101 in the heating channel 100, the pumps of the apparatus, and other functions of the dissolution testing apparatus as well as for recording the testing conditions.

Figures 8a and 8b illustrate a testing method provided by the dissolution testing apparatus of the present invention in an open loop configuration and a closed loop configuration using a flow-through cell 4.

In the open loop configuration shown in Figure 8a, fresh dissolution medium is supplied by one or more medium reservoirs 15 provided by the media selector 80 and pumped towards the flow-through cell 4 by the medium pump 60. The dissolution medium flows through the heating module 6, where it is heated to a pre-selected temperature, and into the flow-through cell 4 in the heating channel 100. The dissolution medium flows through the flow-through cell 4 and crosses the dissoluble substance 5 to create a sample solution. The sample solution flows from the flow-through cell to the bypass valve 32, which directs the sample solution to the extraction module 2 or to a waste conduit 61. In the extraction module 2 fractions of the sample solution are extracted into sample vials 8 for testing. The total amount of dissolution medium is determined by the flow rate created by the medium pump. In the open loop configuration, the total media volume is infinite. The dissolution medium heating module 6 and the heating channel 100 according to the present invention can be used advantageously to temper the flow of fresh dissolution medium. Over time the dissoluble substance diminishes and less substance dissolves into the medium. Therefore, the measured dissolution is represented by a differential curve over time showing decreasing dissolution rates.

The closed loop configuration as shown in Figure 8b works with a fixed volume of dissolution medium supplied by a dissolution medium reservoir 15. The dissolution medium is re-circulated through the flow-through cell 4 and across the dissoluble substance several times. A testing device 70, like a UV spectrophotometer, can be arranged online for testing the sample solution while it circulates in the apparatus. Alternatively, small defined samples can be extracted for testing while the majority of sample solution is re-circulated in the closed loop system. The measured dissolution is represented by a cumulative curve over time, wherein the dissolved substance accumulates in the fixed medium volume over several cycles.

In summary, the dissolution testing apparatus according to the present invention facilitates accurate and reproducible temperature conditions for simulating in vivo conditions for dissoluble substance of all forms as listed in the beginning. The dissolution testing apparatus allows for easy change of cell types without the need of changing the heating system of the apparatus. The dissolution medium heating modules and the heating channel in the dissolution module can be used for all types of dissolution cells. The dissolution testing apparatus enables versatile and automated dissolution testing and supports precise and predictive test results.

The dissolution testing apparatus according to the present invention allows for precise and repeatable measurements of the dissolution rate and dissolution amount of a dissoluble substance of a formulation, which is important for product safety and efficacy. The dissolution testing apparatus facilitates quality control and formulation optimization, it enables accurate prediction of in vivo drug release profiles and reliable assessments of batch-to-batch consistency. Further, it plays an important role in product compliance and market release decisions.

### List of Reference Numbers

| | | | |
|---|---|---|---|
| 1 | dissolution module | 30 | bypass valve |
| 2 | extraction module | 31 | bypass conduit |
| 3 | sample module | 32 | bypass valve |
| 4 | flow-through cell | 33 | temperature sensor |
| 5 | dissoluble substance | 34 | temperature sensor |
| 6 | dissolution medium heating module | 35 | pressure gauge |
| | | 40 | in-flow conduit |
| 7 | sample solution conduit | 41 | out-flow conduit |
| 8 | sample vial | 42 | cleaning conduit |
| 10 | cell volume | 50 | closing ring |
| 11 | cell chamber | 51 | filter |
| 12 | cell inlet | 52 | cap |
| 13 | cell outlet | 60 | medium pump |
| 14 | heating loop structure | 61 | waste conduit |
| 15 | medium reservoir | 70 | testing device |
| 16 | medium inlet | 80 | media selector |
| 17 | medium outlet | 90 | assembly structure |
| 18 | heating element | 91 | cell rack holder |
| 21 | through hole | 92 | connector bar |
| 22 | through hole | 93 | connector platform |
| 23 | heating block | | |
| 26 | pressure sensor | 100 | heating channel |
| 27 | medium inlet line | 101 | heater |
| 28 | medium outlet line | 102 | first elongated section |
| | | 103 | second elongated section |
| | | 104 | ventilator |
| | | 105 | guiding plates |
| | | 106 | temperature sensor |

## Claims

1. Dissolution medium heating module for a dissolution testing apparatus for dissolving a dissoluble substance in the dissolution medium, wherein the heating module (6) comprises a heating chamber, which is connected or connectable to a dissolution medium reservoir (15) for receiving the dissolution medium, a heating module housing comprising fluid connectors, and a heater arrangement for heating the dissolution medium in the heating chamber,
**characterized in that:**
the heating chamber is realized as a two-dimensional heating loop structure (14) designed as a medium conduit which is looping in one coordinate plane and comprising a medium inlet (16) at one end of the heating loop structure (14) and a medium outlet (17) at an opposite end of the heating loop structure (14) for receiving and dispensing dissolution medium, and
the heater arrangement comprises at least one planar heating surface (18), and
the heating loop structure (14) and the planar heating surface (18) are arranged in the heating module housing and adjacent to each other.

2. Dissolution medium heating module according to claim 1, wherein the medium conduit loops in is said coordinate plane along the at least one planar heating surface (18).

3. Dissolution medium heating module according to claim 1 or 2, wherein the at least one planar heating surface (18) covers an entire area of the two-dimensional heating loop structure (14) in the coordinate plane in which the two-dimensional heating loop structure (14) loops.

4. Dissolution medium heating module according to one of the preceding claims, wherein the at least one planar heating surface (18) is a heating plate, preferably a ceramic plate, comprising interior heating coils.

5. Dissolution medium heating module according to one of the preceding claims, wherein a media selector (80) is connected to the medium inlet (16) of the heating loop structure (14) for selectively adding a dissolution medium.

6. Dissolution medium heating module according to one of the preceding claims, wherein a pressure sensor (26) is arranged in a medium inlet line (27) to the heating loop structure (14) for controlled discharge of dissolution medium to the heating loop structure (14).

7. Dissolution medium heating module according to one of the preceding claims, wherein at least one temperature sensor (33, 34) is provided at and/or in the heating loop structure (14) and a control unit controls the heater arrangement.

8. Dissolution testing apparatus comprising at least one dissolution cell (4) arranged for holding a dissoluble substance to be tested, a pump device (60) for adding dissolution medium to the at least one cell, and at least one dissolution medium heating module (6) according to one of the preceding claims, wherein at least one reservoir (15) is in fluid connection with the medium inlet (16) of the heating loop structure (14) and the medium outlet (17) of the heating loop structure (14) is in fluid connection with the at least on cell (4).

9. Dissolution testing apparatus according to claim 8, wherein one dissolution medium heating module (6) is provided for each dissolution cell (4) of the dissolution testing apparatus.

10. Dissolution testing apparatus according to one of the preceding claims 8 or 9, wherein the heating loop structure (14) of the at least one heating module (6) is arranged in-line of a dissolution medium flow between the at least one reservoir (15) and the at least one (4) cell.

11. Dissolution testing apparatus according to one of the preceding claims 8 to 10, wherein the at least one heating module (6) is detachably connected to a cell inlet (12) and the reservoir (15).

12. Dissolution testing apparatus according to one of the preceding claims 8 to 11, wherein a bypass conduit (31) is arranged between the heating module medium outlet (17) and an outlet (13) of the cell (4) bypassing the cell.

13. Dissolution testing apparatus according to one of the preceding claims 8 to 12, wherein the at least one cell (4) is a flow-through cell comprising a cell inlet (12) for the dissolution medium and a cell outlet (13) for dissolution medium containing dissolved substance.

14. Dissolution testing apparatus according to one of the preceding claims 8 to 13, wherein the at least one cell is arranged in a heating channel (100) and a heater (101) is connected to the heating channel (100) for heating a gaseous heating medium in the heating channel (100).

## Patentansprüche

1. Auflösungsmediumheizmodul für eine Auflösungsprüfeinrichtung zum Auflösen einer auflösbaren Substanz in dem Auflösungsmedium, wobei das Heizmodul (6) eine Heizkammer, die mit einem Auflösungsmediumreservoir (15) zum Aufnehmen des Auflösungsmediums verbunden oder verbindbar ist, ein Heizmodulgehäuse, umfassend Fluidverbinder, und eine Heizanordnung zum Erhitzen des Auflösungsmediums in der Heizkammer umfasst,
**dadurch gekennzeichnet, dass**:
die Heizkammer als eine zweidimensionale Heizwindungsstruktur (14) realisiert ist, die als ein sich in einer Koordinatenebene windendes Mediumleitungsrohr gestaltet ist, und umfassend einen Mediumeinlass (16) an einem Ende der Heizwindungsstruktur (14) und einen Mediumauslass (17) an einem gegenüberliegenden Ende der Heizwindungsstruktur (14) zum Aufnehmen und Abgeben des Auflösungsmediums, und
die Heizanordnung mindestens eine ebene Heizoberfläche (18) umfasst, und
die Heizwindungsstruktur (14) und die ebene Heizoberfläche (18) in dem Heizmodulgehäuse angeordnet sind und aneinander angrenzen.

2. Auflösungsmediumheizmodul nach Anspruch 1, wobei das Mediumleitungsrohr sich in der Koordinatenebene entlang der mindestens einen ebenen Heizoberfläche (18) windet.

3. Auflösungsmediumheizmodul nach Anspruch 1 oder 2, wobei die mindestens eine ebene Heizoberfläche (18) einen gesamten Bereich der zweidimensionalen Heizwindungsstruktur (14) in der Koordinatenebene abdeckt, in der sich die zweidimensionale Heizwindungsstruktur (14) windet.

4. Auflösungsmediumheizmodul nach einem der vorstehenden Ansprüche, wobei die mindestens eine ebene Heizoberfläche (18) eine Heizplatte, vorzugsweise eine Keramikplatte, umfassend innere Heizschlangen, ist.

5. Auflösungsmediumheizmodul nach einem der vorstehenden Ansprüche, wobei ein Medienwähler (80) mit dem Mediumeinlass (16) der Heizwindungsstruktur (14) zum selektiven Hinzufügen eines Auflösungsmediums verbunden ist.

6. Auflösungsmediumheizmodul nach einem der vorstehenden Ansprüche, wobei ein Drucksensor (26) in einer Mediumeinlassleitung (27) zu der Heizwindungsstruktur (14) für eine gesteuerte Ausgabe des Auflösungsmediums an die Heizwindungsstruktur (14) angeordnet ist.

7. Auflösungsmediumheizmodul nach einem der vorstehenden Ansprüche, wobei mindestens ein Temperatursensor (33, 34) an und/oder in der Heizwindungsstruktur (14) bereitgestellt ist und eine Steuereinheit die Heizanordnung steuert.

8. Auflösungsprüfeinrichtung, umfassend mindestens eine Auflösungszelle (4), die zum Halten einer zu prüfenden auflösbaren Substanz angeordnet ist, eine Pumpenvorrichtung (60) zum Hinzufügen des Auflösungsmediums zu der mindestens einen Zelle, und mindestens ein Auflösungsmediumheizmodul (6) nach einem der vorstehenden Ansprüche, wobei mindestens ein Reservoir (15) in Fluidverbindung mit dem Mediumeinlass (16) der Heizwindungsstruktur (14) steht und der Mediumauslass (17) der Heizwindungsstruktur (14) in Fluidverbindung mit der mindestens einen Zelle (4) steht.

9. Auflösungsprüfeinrichtung nach Anspruch 8, wobei ein Auflösungsmediumheizmodul (6) für jede Auflösungszelle (4) der Auflösungsprüfeinrichtung bereitgestellt ist.

10. Auflösungsprüfeinrichtung nach einem der vorstehenden Ansprüche 8 oder 9, wobei die Heizwindungsstruktur (14) des mindestens einen Heizmoduls (6) in einer Linie mit einem Fluss des Auflösungsmediums zwischen dem mindestens einen Reservoir (15) und der mindestens einen (4) Zelle angeordnet ist.

11. Auflösungsprüfeinrichtung nach einem der vorstehenden Ansprüche 8 bis 10, wobei das mindestens eine Heizmodul (6) mit einem Zelleneinlass (12) und dem Reservoir (15) lösbar verbunden ist.

12. Auflösungsprüfeinrichtung nach einem der vorstehenden Ansprüche 8 bis 11, wobei ein Umgehungsleitungsrohr (31) zwischen dem Mediumauslass (17) des Heizmoduls und einem Auslass (13) der Zelle (4) der Zelle angeordnet ist, wobei es die Zelle umgeht.

13. Auflösungsprüfeinrichtung nach einem der vorstehenden Ansprüche 8 bis 12, wobei die mindestens eine Zelle (4) eine Durchflusszelle ist, umfassend einen Zelleneinlass (12) für das Auflösungsmedium und einen Zellenauslass (13) für das Auflösungsmedium, das eine aufgelöste Substanz enthält.

14. Auflösungsprüfeinrichtung nach einem der vorstehenden Ansprüche 8 bis 13, wobei die mindestens eine Zelle in einem Heizkanal (100) angeordnet ist und ein Heizer (101) mit dem Heizkanal (100) zum Erhitzen eines gasförmigen Heizmediums in dem Heizkanal (100) verbunden ist.

## Revendications

1. Module de chauffage de milieu de dissolution destiné à un appareil d'essai de dissolution permettant de dissoudre une substance dissoluble dans le milieu de dissolution, dans lequel le module de chauffage (6) comprend une chambre de chauffage, qui est raccordée ou raccordable à un réservoir de milieu de dissolution (15) permettant de recevoir le milieu de dissolution, un logement de module de chauffage comprenant des raccords de fluide, et un agencement de chauffage permettant de chauffer le milieu de dissolution dans la chambre de chauffage,
**caractérisé en ce que** :
la chambre de chauffage est réalisée en tant que structure de boucle de chauffage (14) bidimensionnelle conçue en tant que conduit de milieu qui fait une boucle dans un plan de coordonnées et comprenant une entrée de milieu (16) au niveau d'une extrémité de la structure de boucle de chauffage (14) et une sortie de milieu (17) au niveau d'une extrémité opposée de la structure de boucle de chauffage (14) permettant de recevoir et de distribuer un milieu de dissolution, et
l'agencement de chauffage comprend au moins une surface de chauffage plane (18), et
la structure de boucle de chauffage (14) et la surface de chauffage plane (18) sont agencées dans le logement de module de chauffage et sont adjacentes l'une à l'autre.

2. Module de chauffage de milieu de dissolution selon la revendication 1, dans lequel le conduit de milieu fait une boucle dans ledit plan de coordonnées le long de l'au moins une surface de chauffage plane (18).

3. Module de chauffage de milieu de dissolution selon la revendication 1 ou 2, dans lequel l'au moins une surface de chauffage plane (18) couvre une aire entière de la structure de boucle de chauffage bidimensionnelle (14) dans le plan de coordonnées dans lequel la structure de boucle de chauffage (14) bidimensionnelle fait une boucle.

4. Module de chauffage de milieu de dissolution selon l'une des revendications précédentes, dans lequel l'au moins une surface de chauffage plane (18) est une plaque chauffante, de préférence une plaque en céramique, comprenant des bobines de chauffage intérieures.

5. Module de chauffage de milieu de dissolution selon l'une des revendications précédentes, dans lequel un sélecteur de milieu (80) est raccordé à l'entrée de milieu (16) de la structure de boucle de chauffage (14) permettant d'ajouter sélectivement un milieu de dissolution.

6. Module de chauffage de milieu de dissolution selon l'une des revendications précédentes, dans lequel un capteur de pression (26) est agencé dans une ligne d'entrée de milieu (27) vers la structure de boucle de chauffage (14) pour une décharge commandée de milieu de dissolution vers la structure de boucle de chauffage (14).

7. Module de chauffage de milieu de dissolution selon l'une des revendications précédentes, dans lequel au moins un capteur de température (33, 34) est fourni au niveau de et/ou dans la structure de boucle de chauffage (14) et une unité de commande commande l'agencement de chauffage.

8. Appareil d'essai de dissolution comprenant au moins une cellule de dissolution (4) agencée pour contenir une substance dissoluble à tester, un dispositif de pompe (60) permettant d'ajouter un milieu de dissolution à l'au moins une cellule, et au moins un module de chauffage de milieu de dissolution (6) selon l'une des revendications précédentes, dans lequel au moins un réservoir (15) est en communication fluidique avec l'entrée de milieu (16) de la structure de boucle de chauffage (14) et la sortie de milieu (17) de la structure de boucle de chauffage (14) est en communication fluidique avec l'au moins une cellule (4).

9. Appareil d'essai de dissolution selon la revendication 8, dans lequel un module de chauffage de milieu de dissolution (6) est fourni pour chaque cellule de dissolution (4) de l'appareil d'essai de dissolution.

10. Appareil d'essai de dissolution selon l'une des revendications 8 ou 9 précédentes, dans lequel la structure de boucle de chauffage (14) de l'au moins un module de chauffage (6) est agencée en ligne d'un écoulement de milieu de dissolution entre l'au moins un réservoir (15) et l'au moins une (4) cellule.

11. Appareil d'essai de dissolution selon l'une des revendications 8 à 10 précédentes, dans lequel l'au moins un module de chauffage (6) est raccordé de façon détachable à une entrée de cellule (12) et au réservoir (15).

12. Appareil d'essai de dissolution selon l'une des revendications 8 à 11 précédentes, dans lequel un conduit de contournement (31) est agencé entre la sortie de milieu (17) de module de chauffage et une sortie (13) de la cellule (4) contournant la cellule.

13. Appareil d'essai de dissolution selon l'une des revendications 8 à 12 précédentes, dans lequel l'au moins une cellule (4) est une cellule à écoulement traversant comprenant une entrée de cellule (12) pour le milieu de dissolution et une sortie de cellule (13) pour le milieu de dissolution contenant une substance dissoute.

14. Appareil d'essai de dissolution selon l'une des revendications 8 à 13 précédentes, dans lequel l'au moins une cellule est agencée dans un canal de chauffage (100) et un chauffage (101) est raccordé au canal de chauffage (100) pour chauffer un milieu chauffant gazeux dans le canal de chauffage (100).
